# EUROPEAN PATENT SPECIFICATION

(11) **EP 0 575 189 B1**
(45) Date of publication and mention of the grant of the patent: **12.03.1997**
(21) Application number: 93304760.7
(22) Date of filing: 17.06.1993
(51) Int. Cl.: C07C 53/10, C07C 53/122, C07C 53/124, C07C 51/41, C07C 63/08

(54) **Organic cerium (IV) compounds and their preparation and use**
Organische Cerium-IV-Verbindungen und deren Herstellung und Verwendung
Composés de cérium IV organiques et leur préparation et utilisation

(30) Priority: 17.06.1992 GB 9212811; 23.09.1992 GB 9220116
(43) Date of publication of application: 22.12.1993
(73) Proprietor: RHONE-POULENC CHEMICALS LIMITED, Watford, Herts WD1 1QM (GB)
(72) Inventor: Hawkins, Ian Michael, Saddleworth, Oldham, Lancashire, OL3 7JG (GB); Mauermann, Heiko Heinrich Karl, Plainsboro, New Jersey 08536 (US)
(74) Representative: Woods, Geoffrey Corlett

(56) References cited:
- EP-A- 0 433 133
- GB-A- 1 475 971

## Description

The present invention relates to cerium (IV) compounds and their preparation and use and more particularly the present invention provides novel cerium (IV) derivatives which are soluble in organic solvents including more particularly hydrocarbons, or are convertible into cerium (IV) compounds which are soluble in hydrocarbons.

Cerium acts as a catalyst in numerous applications. More particularly, it can be used to catalyse the crosslinking of film-forming compositions which dry by atmospheric oxidation, e.g. paint films, and it has been proposed as a catalyst in the oxidation of combustion residues of hydrocarbon fuels, e.g. diesel fuels as disclosed in USP 4522631.

For these different uses, it is important to be able to obtain cerium compounds in the cerium (IV) valency state, which is often the catalytically active state, which are soluble in organic solvents, stable, and capable of being stored at high cerium concentrations.

In United States Patent No. 4599201 (Gradeff et al) and the corresponding European specification EP-A-0093627, it has been proposed to make ceric carboxylates which are soluble in organic solvents from a solution of a cerium (III) compound which is oxidised in an organic medium. Although this process has advantages, it has also the following disadvantages:
(1) the products obtained are ill defined and in particular contain a mixture of cerium (III) and cerium (IV) in which the proportion of Ce (IV) is not above 75%;
(2) the solutions obtained are extremely viscous and their long term stability is doubtful;
(3) it involves the use of hydrogen peroxide; and
(4) some cerium is lost in the form of water-soluble byproducts.

The present invention provides novel organic solvent soluble cerium (IV) compounds which avoid these disadvantages, and have improved solubility in organic solvents. More particularly, the present invention provides cerium (IV) compounds containing, for each Ce(IV) atom, two residues of an organic acid having a pKa greater than 1, or of a mixture of such acids, and one oxygen atom bound to said Ce (IV) atom additional to the oxygen atoms in the said organic acid residues, and obtainable by reaction, preferably in aqueous solution, of a cerium (IV) salt with a salt of a said organic acid having a pKa greater than 1, or with a mixture of salts of organic acids at least one of which has a pKa greater than 1, using at least two molar proportions of said salts of the organic acid per atom of cerium.

The organic acid is preferably a carboxylic acid but may be an organic sulphuric, sulphonic, phosphonic, or phosphoric acid of the required pKa. These novel cerium (IV) compounds are storage stable in the pure state and also when dissolved in a hydrocarbon solvent, and the cerium (IV) content is at least 90%, usually at least 95%, and often at least 99% of the total cerium content.

The new cerium (IV) compounds are obtained as yellow crystalline solids or yellow liquids. It is believed that they may be represented by the formula

(H₂O)ₚ[CeO(A)₂.(AH)ₙ.]ₘ

where the A radicals are the same or different and each is the residue of an organic acid as defined above of formula AH, p is an integer from 0 to 5, n is from 0 to 2 and m is an integer from 1 to 12. More particularly, n is preferably 0 or 2, p is 4, and m is 6. The cerium (IV) compounds containing the organic acid residues in which n is greater than 0 (preferably 2) are liquids which are miscible with organic hydrocarbon solvents. When n is 0 the compounds are crystalline solids which, if not soluble in hydrocarbon solvents, may be made soluble by reaction with an acid, or a mixture of acids, to produce a product in which n is greater than 0, e.g. 2.

X-ray studies of the new products, when crystallized, have shown that at least in some cases the cerium atoms are arranged at the apexes of an octahedron with each of the eight faces capped by a triply bridging oxygen atom, and twelve carboxylic acid residues arranged in bidentate configuration along the edges of the octahedron. In addition two unidentate oxygen atoms, believed to be in hydroxyl groups, are attached to two opposite cerium atoms of the Ce₆ octahedron. To judge from the infrared spectra obtained for solutions of the new cerium (IV) compounds, this structure is largely conserved in the organic solvent solutions and in the liquid cerium (IV) carboxylates.

Preferred such compounds may be represented by the formula:

H₆Ce₆O₈(OH)₂(RCOO)₁₂

where R is alkyl of 3 to 9 carbon atoms, having an octahedral structure with the six cerium atoms at the apexes of the octahedron, the twelve carboxylate residues forming bidentate bridges between the cerium atoms along the edges of the octahedron, one triply bridging oxygen atom on each face of the octahedron and two unidentate hydroxyl ligands completing the coordination of two opposite cerium atoms.

According to a feature of the invention, the novel cerium (IV) compounds are obtained by mixing, preferably in an aqueous reaction medium, a cerium (IV) salt with a salt of an organic acid having a pKa greater than 1 or with a mixture of salts of organic acids at least one of which has a pKa greater than 1 (which may be formed in situ) using at least two molecular proportions of said salt of an organic acid per atom of cerium.

The organic acid salt is preferably prepared by reaction of the organic acid with an alkali metal, alkaline earth metal or ammonium (preferably tetra(lower alkyl) ammonium) oxide, hydroxide, carbonate or hydrogen carbonate immediately before use, or in situ in the presence of the cerium (IV) salt.

It has been found that it is highly preferable to use at least four molecular proportions of the organic acid salt per atom of cerium in order to obtain complete reaction. It is believed that this is because the initial product of the reaction is an intermediate containing four acid residues per cerium atom. This intermediate then hydrolyses to produce the products in accordance with the present invention having, for each cerium atom, an oxygen atom and two acid residues together with associated hydroxyl or water ligands.

The cerium (IV) salt used is preferably water soluble and may be for example a nitrate or sulphate. Preferably it is ceric ammonium nitrate. The cerium (IV) salt may also be reacted with the salt of the organic acid in an organic solvent, especially a highly polar organic solvent.

It is also possible to use basic cerium (IV) compounds in suspension, e.g. cerium (IV) carbonate, cerium (IV) oxycarbonate, or cerium (IV) hydroxycarbonate. Such basic cerium (IV) compounds may be reacted with four moles of organic acid per mole of cerium, in the absence of any additional added base.

The salt of the organic acid is preferably an alkali metal salt such as a sodium or potassium salt or an ammonium salt.

The reaction may be carried out at any temperature between 0°C and the boiling point of the reaction mixture, e.g. between 10° and 80°C, but is preferably operated at ambient temperature, e.g. 20°C up to 30°C.

The salt of the organic acid is preferably prepared in situ by reaction of the acid with an alkali metal, alkaline earth metal, or ammonium oxide, hydroxide, hydrogen carbonate or carbonate, e.g. sodium hydroxide or ammonium hydrogen carbonate, to a pH in the range of 7 to 10, preferably about 8.

When an aqueous reaction medium is used, the concentration of the cerium (IV) salt is conveniently in the range from 0.1 to 2.0 molar. This solution may be added to the preformed solution of the organic acid salt in a concentration which is preferably 0.5 to 2 molar.

The acids which may be used in the present invention are in general organic acids having a pKa greater than 1, and usually greater than 2, preferably carboxylic acids containing at least 2 carbon atoms, and usually alkanoic acids of 4 to 20, preferably 4 to 12, carbon atoms, which may be straight or branched chain. Dicarboxylic acids of 4 to 12 carbon atoms, or aromatic or arylaliphatic acids such as benzoic acid which may be substituted by alkyl of up to 12 carbon atoms, can also be used. These acids may be used individually or in admixture.

While increasing the number of carbon atoms increases the solubility of the cerium (IV) compounds in hydrocarbon solvents, it also reduces the concentration of cerium obtainable in the solution. It is therefore advantageous that the average number of carbon atoms present in the acid or mixture of acids used should preferably be in the range of 3 to 15 and preferably 4 to 8.

Examples of acids which are suitable for use in the present invention include acetic acid, propionic acid, butyric acid, isobutyric acid (2-methyl-propionic acid), pivalic acid, 2-methyl-butyric acid, 2,2-dimethylbutyric acid, 2-ethylbutyric acid, 2-methyl-pentanoic acid, 2-ethylhexanoic acid (octanoic or octoic acid), ethylacetoacetic acid, 3,5-dimethylhexanoic acid, cyclohexane carboxylic acid, neohexanoic acid (2,2-dimethylbutyric acid), neoheptanoic acid (2,2-dimethyl-pentanoic acid), neooctanoic acid, isononanic acid (cekanoic acid), neodecanoic acid, undecylenic acid, perfluorobutyric acid, benzoic acid, p-tert-butyl-benzoic acid, naphthenic acid, anthranilic acid, behenic acid, maleic acid, sebacic acid, bis(2-ethylhexyl)phosphoric acid, dodecylbenzene sulphonic acid, dodecylsulphuric acid, p-toluene sulphonic acid and their mixtures.

The acids may be straight or branched chain. Carboxylic acids substituted in the alpha or beta position in relation to the carboxylic acid group often form cerium (IV) derivatives having an advantageous degree of solubility, especially when there is only a single substituent, preferably containing not more than four carbon atoms.

The acids should be chosen so as to be stable in the reaction medium used to make the new compounds. The organic acid may be substituted, e.g. by halogen, hydroxy or oxo radicals, as in pyruvic acid, alpha-hydroxy carboxylic acids, and keto acids such as aceto acetic acid. They may also contain ether or ester groups. Since cerium (IV) is a strong oxidizing agent, it is advisable to avoid the use of carboxylic acids which are easily oxidized, e.g. because they contain several double bonds or other oxidizable groups.

The cerium (IV) compounds in accordance with the present invention are surprisingly soluble in apolar solvents, e.g. hydrocarbons, but also in polar solvents, e.g. carboxylic acids, ethers, alcohols and halogenated hydrocarbons. The preferred cerium (IV) compounds for dissolution, or dilution, in hydrocarbons are those in which n is from 0 to 2 and/or the total number of carbon atoms in the A radicals is at least 10, and preferably at least 24, per atom of cerium. For liquid products of high cerium content the total number of carbons per atom of cerium should not exceed 60. The structure of the carboxylate ligand is believed to be important in determining the stoichiometry and organosolubility of the ceric carboxylate product. Results for homoleptic (single ligand) complexes are tabulated below.

| CARBOXYLATE CHAIN STRUCTURE | MINIMUM CARBON ATOM CONTENT OF ACID FOR PRODUCT TO BE: | |
|---|---|---|
| | A. HYDROCARBON MISCIBLE LIQUID | B. HYDROCARBON SOLUBLE SOLID |
| 2-methyl branched | C₆ | C₅ |
| 2-ethyl branched | C₈ | C₆ |
| 2,2-dimethyl branched | C₁₀ | C₆,C₇ |
| chain branched | C₈ | |
| linear | | >C₁₀ |

Examples of specific cerium (IV) compounds include
Cerium (IV) Acetate Octoate^{L}
Cerium (IV) Anthranilate Octoate
Cerium (IV) Behenate
Cerium (IV) Benzoate
Cerium (IV) Benzoate Octoate
Cerium (IV) bis(2-ethylhexyl)phosphate^{L}
Cerium (IV) bis(2-ethylhexyl)phosphate Octoate^{L}
Cerium (IV) Butyrate
Cerium (IV) Butyrate Octoate^{L}
Cerium (IV) Cekanoate^{L}
Cerium (IV) Cyclohexane carboxylate^{L}
Cerium (IV) 2,2-dimethylbutyrate
Cerium (IV) Dodecylsulphate
Cerium (IV) Dodecylsulphate Octoate
Cerium (IV) Dodecylbenzenesulphonate
Cerium (IV) Dodecylbenzenesulphonate Octoate^{L}
Cerium (IV) 2-ethylbutyrate^{S}
Cerium (IV) 2-ethylbutyrate Octoate^{L}
Cerium (IV) Isobutyrate
Cerium (IV) Isobutyrate Octoate^{L}
Cerium (IV) Isobutyrate_{3.0}Octoate_{1.0}^{S}
Cerium (IV) Maleate
Cerium (IV) Maleate Octoate
Cerium (IV) 2-methylbutyrate^{S}
Cerium (IV) 2-methylbutyrate Octoate^{L}
Cerium (IV) 2-methylpentanoate^{L}
Cerium (IV) Naphthenate^{L}
Cerium (IV) Neodecanoate^{L}
Cerium (IV) Neoheptanoate^{S}
Cerium (IV) Neooctanoate^{L}
Cerium (IV) Octoate^{L}
Cerium (IV) (Octoate)₃(EAA)^{L}
Cerium (IV) Perfluorobutyrate
Cerium (IV) Pivalate
Cerium (IV) Pivalate Octoate^{L}
Cerium (IV) Propionate
Cerium (IV) Propionate Octoate^{S}
Cerium (IV) p-tertbutylbenzoate^{S}
Cerium (IV) p-tertbutylbenzoate Octoate^{L}
Cerium (IV) p-toluenesulphonate Octoate
Cerium (IV) Sebacate
Cerium (IV) Sebacate Octoate
Cerium (IV) Undecylenate^{S}
Cerium (IV) Undecylenate Octoate^{L}
where L denotes a hydrocarbon soluble liquid
and S denotes a hydrocarbon soluble solid.

The new cerium (IV) compounds may be used in solution or as neat liquids especially in film forming compositions which dry by atmospheric oxidation, e.g. in paints. They may also be used to promote combustion of hydrocarbon fuels. For these purposes they may be dissolved in suitable solvents at the time of use, but it is a significant advantage of the new cerium (IV) compounds that they can easily be made and stored as concentrated liquids having a high cerium (IV) content, e.g. greater than 10 and preferably greater than 20 or even 30% of total weight. Such liquids are characterized inter alia by having, for a given Ce(IV) content, a lower viscosity than Ce(IV)-containing liquids previously known. More particularly a Ce(IV) liquid in accordance with the present invention containing 20% Ce(IV) typically has a viscosity at 20°C of less than 1 poise, e.g. 0.5 to 1 poise. Such liquid cerium (IV) compounds in accordance with the present invention are easily blended into hydrocarbons or compositions based on hydrocarbon solvents (e.g. film forming compositions), with which they are generally miscible and generally in all proportions. They are especially useful as combustion-promoting additives in hydrocarbon fuels, including fuels for internal combustion, especially diesel, engines.

The following Examples illustrate the invention.

In the Examples the acid or mixture of acids (4.0 mol proportions) is converted into its alkali metal, alkaline earth metal or ammonium salt by reaction with the stoichiometric quantity of an appropriate oxide, hydroxide, hydrogen carbonate or carbonate, preferably a hydrogen carbonate, and preferably ammonium hydrogen carbonate. The salt thus formed is added to an aqueous solution of a cerium (IV) salt, preferably ceric ammonium nitrate (1.0 mol proportions) at ambient temperature with stirring. The desired product separates out as a solid or liquid. If it forms a solid, it is filtered off. If it forms a liquid, it is extracted with a hydrocarbon solvent and the organic solution obtained is then separated and washed with water. The washed organic solvent solution is then evaporated, e.g. in a rotary evaporator, to remove the organic solvent. During the evaporation hexane or other suitable solvent is added to remove any residual water by azeotropic distillation. The solvent free product is then obtained as a liquid.

In an alternative preferred procedure, the solid inorganic base is directly added to a stirred two-phase system containing the organic acid and the aqueous ceric ammonium nitrate solution, in a one pot reaction. The organic salt of the acid is formed in situ, and reacts with the aqueous ceric ammonium nitrate solution to give the desired cerium (IV) organic product. Any intermediate cerium (IV) species containing carbonate formed by side reaction of the inorganic base with the aqueous cerium (IV) solution themselves react with the remaining organic acid to yield the same cerium (IV) organic product.

This process can also be carried out in the complete absence of added organic or aqueous reaction medium, but it is highly preferable to dissolve the solid ceric ammonium nitrate at least partly before admixture with the organic acid and subsequent reaction with the inorganic base, especially when the latter is added as a solid.

In Examples 1-22 below the acid or mixture of acids (0.4 mol) was first converted into its sodium salt by reaction with the stoichiometric quantity of sodium hydroxide. The solution was then made up with 300 g H₂O. This is added to a solution of ceric ammonium nitrate (0.1 mol) in water (400 g) at ambient temperature while the mixture was stirred. The desired product separated out as a solid or liquid and was worked up in the manner described above.

The following Table shows the acids used, the yield of the cerium (IV) compound obtained and its form and cerium content.

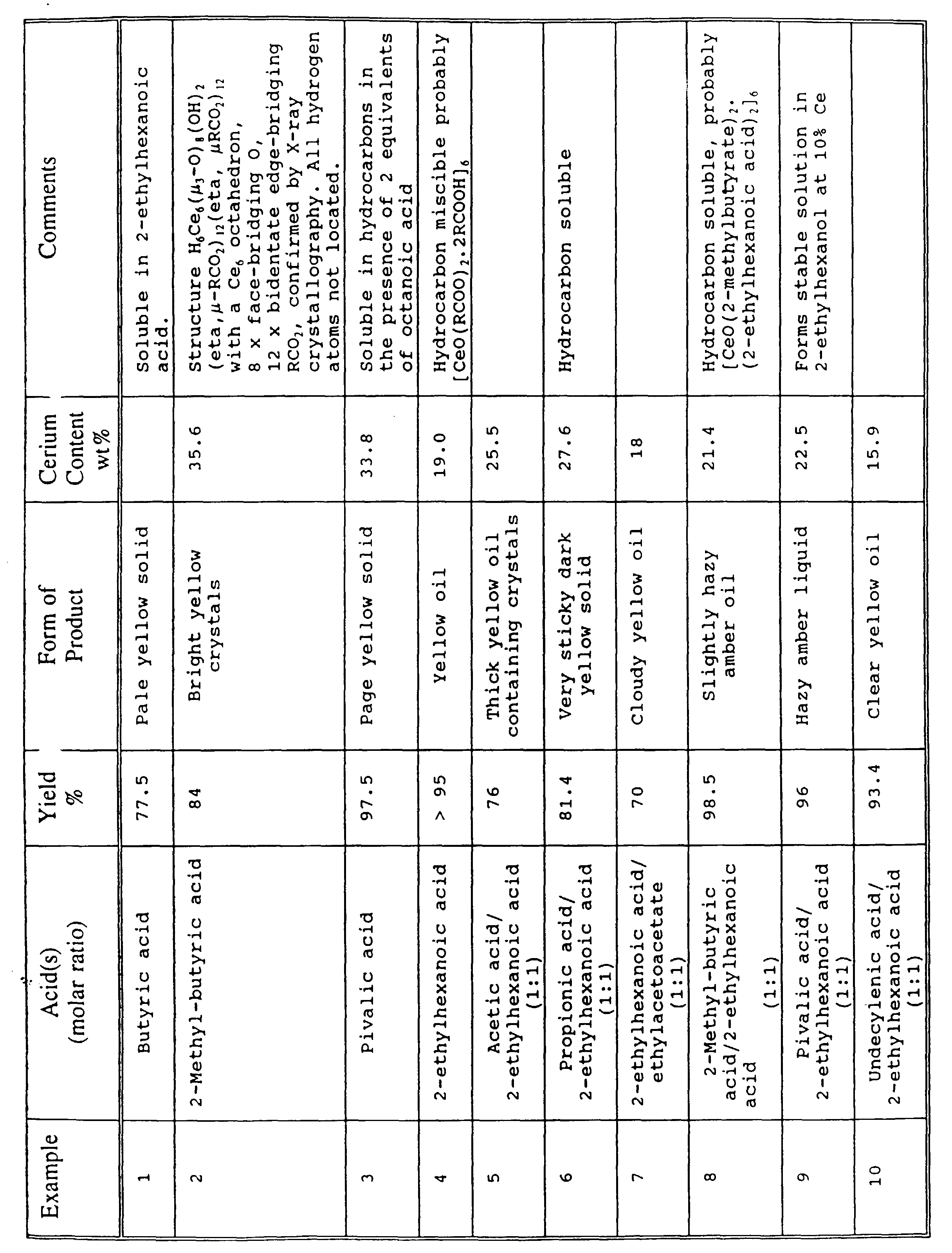

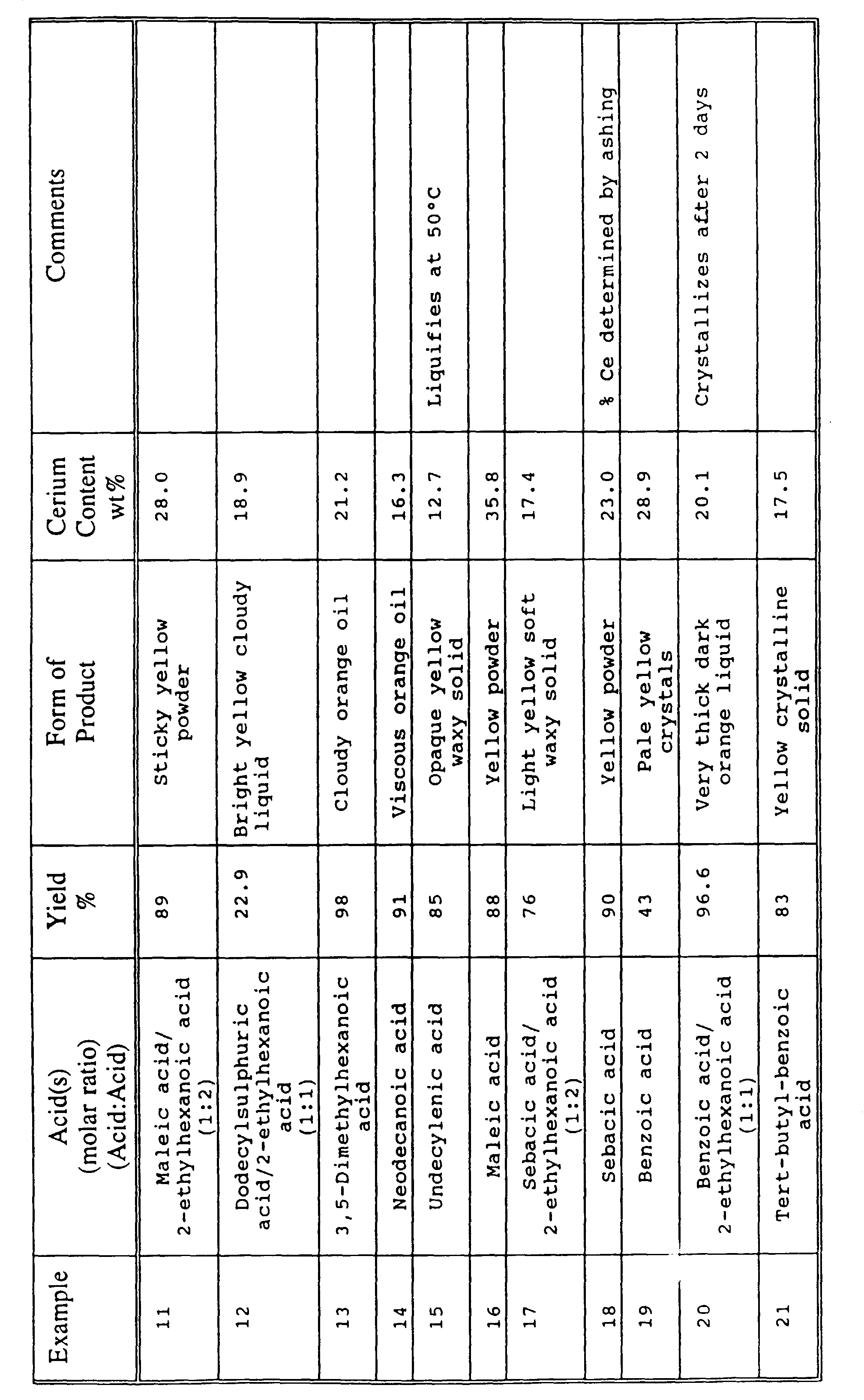

### EXAMPLE 23

The large scale production of a cerium (IV) comound of the invention may be operated as follows: Ceric ammonium nitrate (100 parts by weight, one mole equivalent) is partially dissolved in water (45.45 parts by weight) with stirring. The dissolution is endothermic and an orange solution is obtained. 2-Ethyl-hexanoic acid (104.72 parts by weight, 4 mole equivalents) is added with rapid stirring. The temperature of the mixture is adjusted to 40°C and solid ammonium bicarbonate (57.46 parts by weight, 4 mole equivalents) is then added in portions (each about 5 parts by weight) over a period of 30 minutes. Carbon dioxide is evolved and a yellow oily product forms.

The reaction mixture is kept at about 40°C with continued stirring for a further hour, and then allowed to cool to ambient temperature without stirring. Most of the desired product separates out as a clear, yellow upper organic layer.

Heptane (63.63 parts by weight) is then added with stirring to dissolve the separated product and extract residual product from the aqueous phase. The lower aqueous layer is then removed. The organic layer is washed with water (90 parts by weight) and again separated. The heptane is removed from the organic layer by vacuum distillation at 45-50°C (<50 mm Hg). Any remaining water is removed by distillation with the heptane. The residue is the desired product. It forms a clear bright yellow oil containing 19.0 to 19.5% w/w Ce, of which >95% is Ce(IV), and having a viscosity of about 0.9 poise. The product contains >99% of the cerium initially used.

### Infra-red Spectroscopy

The compounds all share the same major infra-red absorbances, tabulated below:

| BAND | | PROPOSED ASSIGNMENT |
|---|---|---|
| 1. 3650 cm⁻¹ | strong, very sharp | υ OH? υ Ce-O-H? or isolated OH of carboxylic acid |
| 2. 3400-2400cm⁻¹ | strong, very broad | υ OH of free carboxylic acid |
| 3. 1750-1720cm⁻¹ | very strong very sharp | υ C=O of carboxylic acid |
| 4. 1580-1550cm⁻¹ | very strong very sharp | υ C=O of µ, η carboxylate |
| 5. 700-500cm⁻¹ | weak, complex | υ Ce-O of cluster framework |

Comparison of bands 2 and 3 with those of the relevant free acids shows them to be identical. The similarity of the infra-red spectra for all the solid Ce^{IV} carboxylates made to that of Ce^{IV} 2-methylbutyrate suggests that they are isostructural, all containing the Ce₆ core. The variation in intensity of bands 2 and 3 relative to 4 correlates with the higher than theoretical acid to metal ratio found for these complexes, often nearer 2.5:1 than 2:1.

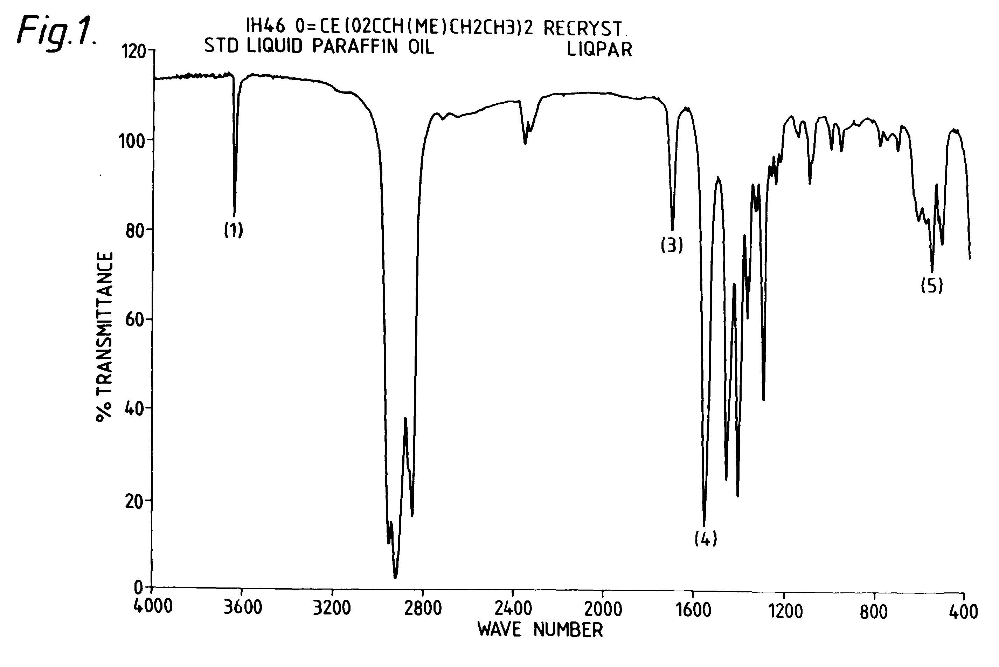

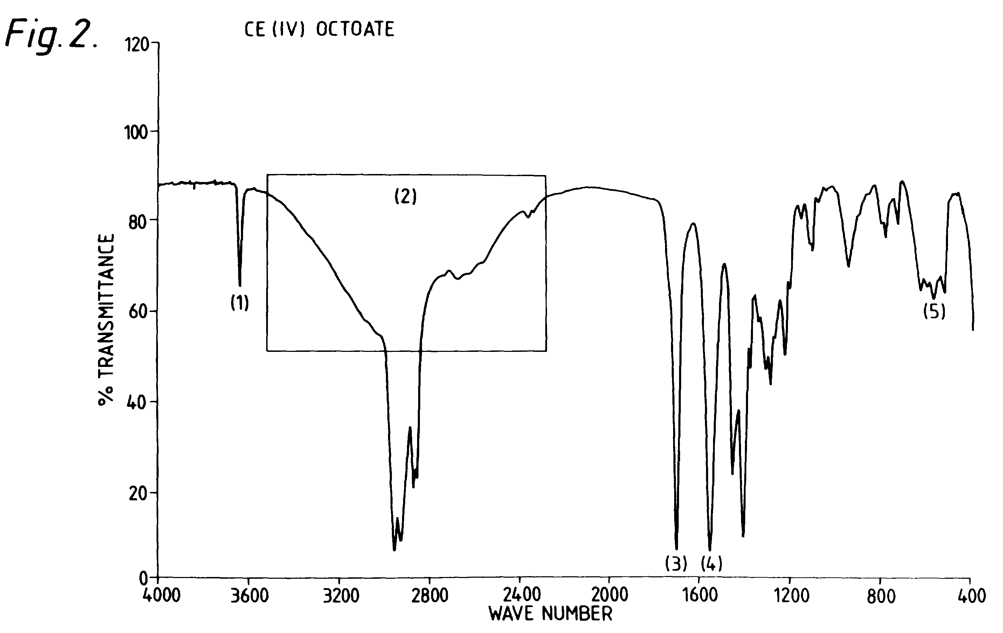

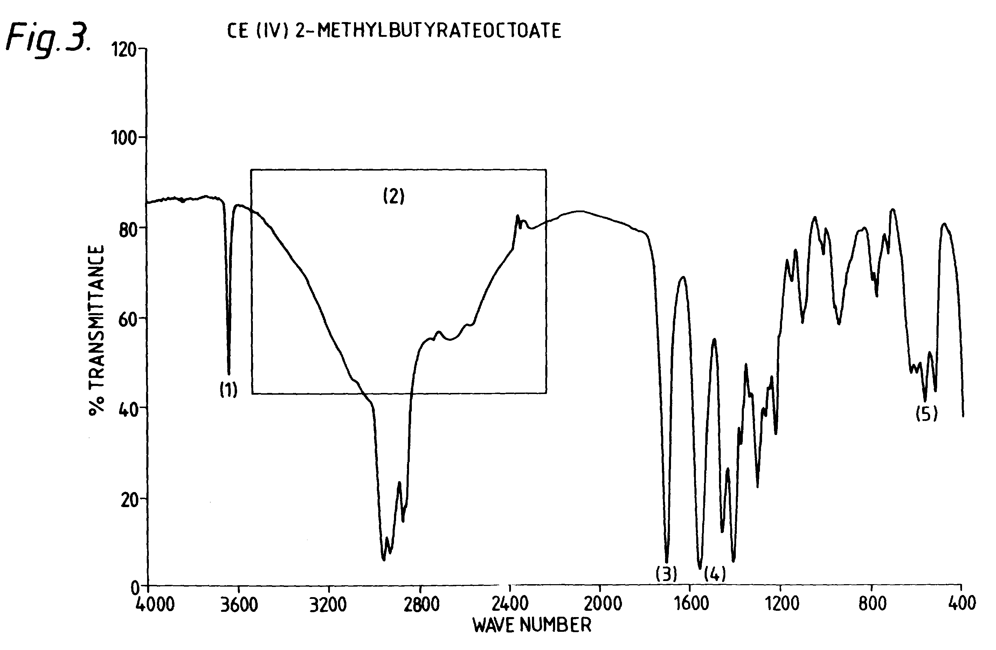

In the accompanying drawings, Figures 1, 2 and 3 show respectively the infra-red absorption spectra of the products of Examples 2 (2-methyl-butyrate), 4(2-ethylhexanoate, i.e. octoate), and 8 (mixed 2-methylbutyrate/octoate). In these spectra, the characteristic absorption bands (1) to (5) noted in the Table above have been indicated.

### Ultraviolet Spectroscopy

All the Ce^{IV} carboxylates made were yellow or orange in colour. However, the UV absorptions giving rise to this colour are weak and hidden beneath the strong metal carboxylate absorbances below 350nm.

Ce^{IV} 2-methylbutyrate shows a weak shoulder to this feature with λₘₐₓ = 350nm and ε = 212.

### EXAMPLE 24

2-Ethyl hexanoic acid (922.88g, 6.4 mol) was placed in a reaction vessel and water (3 litres) was added. Sodium hydroxide solution (50%, 512g, 6.4 mol) was then added rapidly through a dropping funnel. The temperature of the mixture rose to 49°C and the mixture was then heated further to 80°C. The mixture was stirred for 30 minutes until it became clear. The pH was measured with a pH meter and if it was above 8.5, more 2-ethyl hexanoic acid was added to adjust the pH to about 8.5. Ceric ammonium nitrate (904.36g, 1.6 mol) was dissolved in water (2 litres) and the solution was added to the heated solution of sodium 2-ethyl-hexanoate through a dropping funnel over a period of half an hour. A solid sticky material precipitated at first but it dissolved later. The mixture was cooled to ambient temperature and hexane (600g or more if required) was added to extract the product. The organic layer was separated and washed with water 2 to 4 times (400 ml water each time) until the wash water contained no detectable nitrate. If desired, at this stage the aqueous layer from the reaction mixture and the aqueous washes can be combined and re-extracted with more hexane (100 ml), in which case the hexane extract is washed with water to remove nitrate and combined with the main hexane extract.

The hexane solution of the desired product is then filtered if cloudy and hexane is removed by distillation in vacuo at ambient or slightly elevated temperature (below 50°C). The product is an orange oil having a density of 1.2g/ml and a viscosity of 281 cps. The yield was 1120g (92.2%), without re-extraction of the aqueous layer. The product had a cerium content of 18.46%.

## Claims

1. A cerium (IV) compound containing, for each Ce(IV) atom, two residues of an organic acid having a pKa greater than 1, or of a mixture of such acids, and one oxygen atom bound to said Ce (IV) atom additional to the oxygen atoms in the said organic acid residues, and obtainable by reaction of a cerium (IV) salt with a salt of a said organic acid having a pKa greater than 1, or with a mixture of salts of organic acids at least one of which has a pKa greater than one, using at least two molar proportions of said salts of the organic acid per atom of cerium.

2. A cerium (IV) compound of the formula:
(H₂O)ₚ[CeO(A)₂.(AH)ₙ.]ₘ
where the A radicals are the same or different and each is the residue of an organic acid as defined in claim 1 of formula AH, p is an integer from 0 to 5, n is from 0 to 2, and m is an integer from 1 to 12.

3. A cerium (IV) compound according to claim 2 of the formula:
(H₂O)₄[CeO(A)₂.(AH)ₙ.]₆
wherein A is defined in claim 2 and n is 0 or 2.

4. A cerium (IV) compound according to claim 1, 2 or 3 in which the said pKa is greater than 2.

5. A cerium (IV) compound according to any one of claims 1 to 4 in which the organic acid is a carboxylic, organic sulphuric, sulphonic, phosphoric or phosphonic acid.

6. A cerium (IV) compound according to any one of claims 1 to 5 in which the organic acid is a monocarboxylic acid having 2 to 20 carbon atoms, or a dicarboxylic acid having 4 to 12 carbon atoms.

7. A cerium (IV) compound according to any one of claims 1 to 5 in which the organic acid is an alkanoic acid of 4 to 12 carbon atoms, benzoic acid or benzoic acid substituted by alkyl of up to 12 carbon atoms, or a mixture thereof.

8. A cerium (IV) compound according to claim 1, 2 or 3 in which the organic acid is one or more of acetic acid, propionic acid, butyric acid, 2-methyl-propionic acid, pivalic acid, 2-methylbutyric acid, 2-ethyl-butyric acid, 2-methyl-pentanoic acid, 2-ethyl-hexanoic acid, 3,5-dimethylhexanoic acid, neohexanoic acid, neoheptanoic acid, neooctanoic acid, isononanoic acid, neodecanoic acid, undecylenic acid, naphthenic acid, cyclohexane carboxylic acid, benzoic acid, p-tert-butyl-benzoic acid, maleic acid, sebacic acid, bis(2-ethylhexyl)phosphoric acid, dodecylbenzene sulphonic acid and dodecylsulphuric acid.

9. A cerium (IV) compound of the formula:
H₆Ce₆O₈(OH)₂(RCOO)₁₂
where R is alkyl of 3 to 9 carbon atoms, having an octahedral structure with the six cerium atoms at the apexes of the octahedron, the twelve carboxylate residues forming bidentate bridges between the cerium atoms along the edges of the octahedron, one triply bridging oxygen atom on each face of the octahedron and two unidentate hydroxyl ligands completing the coordination of two opposite cerium atoms.

10. A cerium (IV) compound according to any one of the preceding claims showing the major infra-red absorbances tabulated below:
| BAND | | PROPOSED ASSIGNMENT |
|---|---|---|
| 1. 3650 cm⁻¹ | strong, very sharp | υ OH? υ Ce-O-H? or isolated OH of carboxylic acid |
| 2. 3400-2400cm⁻¹ | strong, very broad | υ OH of free carboxylic acid |
| 3. 1750-1720cm⁻¹ | very strong very sharp | υ C=O of carboxylic acid |
| 4. 1580-1550cm⁻¹ | very strong very sharp | υ C=O of µ, η carboxylate |
| 5. 700-500cm⁻¹ | weak, complex | υ Ce-O of cluster framework |

11. A process for the preparation of a cerium (IV) compound as claimed in any of claims 1 to 10 which comprises mixing a cerium (IV) salt with a salt of an organic acid having a pKa greater than 1 or with a mixture of salts of organic acids at least one of which has a pKa greater than 1 using at least 2 molecular proportions of said salt of an organic acid per atom of cerium.

12. A process according to claim 11 in which the cerium (IV) salt is ceric ammonium nitrate.

13. A process according to claim 11 or 12 in which the mixing is effected in an aqueous medium.

14. A solution of a cerium (IV) compound as claimed in any of claims 1 to 10 in a carboxylic acid or a hydrocarbon solvent.

15. A film-forming composition which dries by atmospheric oxidation comprising, as oxidation catalyst, a cerium (IV) compound as claimed in any one of claims 1 to 10.

16. A hydrocarbon fuel comprising an oil-soluble cerium (IV) compound as claimed in any one of claims 1 to 10.

17. A fuel according to claim 16 for an internal combustion engine, especially a diesel fuel.

## Patentansprüche

1. Cer(lV)-Verbindung, die je Cer(lV)-Atom zwei Reste einer organischen Säure mit einem pKa-Wert größer als 1 oder einer Mischung solcher Säuren und neben den Sauerstoffatomen in den genannten Resten der organischen Säure ein Sauerstoffatom enthält, das an das genannte Cer(lV)-Atom gebunden ist und die durch Umsetzung eines Cer(lV)-Salzes mit einem Salz der genannten organischen Säure mit einem pKa größer als 1 oder mit einer Mischung organischer Säuren erhalten werden kann, worunter mindestens eine einen pKa-Wert größer als 1 aufweist, wobei mindestens zwei Mol-Teile der Salze der organischen Säure je Cer-Atom eingesetzt werden.

2. Cer(lV)-Verbindung der Formel :
(H₂O)ₚ[CeO(A)₂.(AH)ₙ.]ₘ
in der die Reste A gleich oder verschieden sind und je den Rest einer organischen Säure der Formel AH gemäß der Definition in Anspruch 1 darstellen, p eine ganze Zahl von 0 bis 5 ist, n 0 bis 2 beträgt und m eine ganze Zahl von 1 bis 12 ist.

3. Cer(lV)-Verbindung nach Anspruch 2 mit der Formel :
(H₂O)₄[CeO(A)₂.(AH)ₙ.]₆
in der A der Definition in Anspruch 2 entspricht und n gleich 0 oder 2 ist.

4. Cer(lV)-Verbindung nach Anspruch 1, 2 oder 3, in der der pKa-Wert größer als 2 ist.

5. Cer(lV)-Verbindung nach einem der Ansprüche 1 bis 4, in der die organische Säure eine Carbonsäure, eine organische Schwefel-, Sulfon-, Phosphor- oder Phosphonsäure ist.

6. Cer(lV)-Verbindung nach einem der Ansprüche 1 bis 5, in der die organische Säure eine Monocarbonsäure mit 2 bis 20 C-Atomen oder eine Dicarbonsäure mit 4 bis 12 C-Atomen ist.

7. Cer(lV)-Verbindung nach einem der Ansprüche 1 bis 5, in der die organische Säure eine Alkansäure mit 4 bis 12 C-Atomen, eine Benzoesäure oder eine alkylsubstituierte Benzoesäure ist, die bis 12 C-Atome aufweisen kann.

8. Cer(lV)-Verbindung nach Anspruch 1, 2 oder 3, in der die organische Säure eine oder mehrere unter folgenden Säuren ist : Essigsäure, Propionsäure, Buttersäure, 2-Methylpropionsäure, Pivalinsäure, 2-Methylbuttersäure, 2-Ethylbuttersäure, 2-Methylpentansäure, 2-Ethylhexansäure, 3,5-Dimethylhexansäure, Neohexansäure, Neoheptansäure, Neooctansäure. Isononansäure, Neodecansäure, Undecylensäure, Naphthensäure. Cyclohexancarbonsäure, Benzoesäure, p-tert-Butylbenzoesäure, Maleinsäure, Sebacinsäure, bis(2-Ethylhexyl)phosphorsäure. Dodecylbenzolsulfonsäure und Dodecylschwefelsäure.

9. Cer(lV)-Verbindung der Formel :
H₆Ce₆O₈(OH)₂(RCOO)₁₂
in der R eine Alkylgruppe mit 3 bis 9 C-Atomen ist,
mit oktaedrischer Struktur, wobei die sechs Cer-Atome an den Spitzen des Oktaeders stehen, die zwölf Carboxylat-Reste zweizähnige Brücken zwischen den Cer-Atomen entlang der Oktaederkanten bilden, ein Sauerstoffatom eine dreifache Brücke an jeder Fläche des Oktaeders bildet und zwei einzähnige Hydroxylliganden die Koordination der beiden gegenüberliegenden Atome vervollständigen.

10. Cer(IV)-Verbindung nach einem der vorhergehenden Ansprüche mit den in der folgenden Tabelle angegebenen infraroten Hauptabsorptionen :
| BANDE | | VORGESCHLAGENE ZUWEISUNG |
|---|---|---|
| 1. 3650 cm⁻¹ | Stark, sehr scharf | υ OH? Ce-O-OH oder einzelne OH-Gruppe des Carbonsäure |
| 2. 3400-2400 cm⁻¹ | Stark, sehr breit | υ OH-Gruppe der freien Carbonsäure |
| 3. 1750-1720 cm⁻¹ | Sehr stark, sehr scharf | υ C=O der Carbonsäure |
| 4. 1580-1550 cm⁻¹ | Sehr stark, sehr scharf | υ C=O des µ, η-Carboxylats |
| 5. 700-500 cm⁻¹ | Schwach, komplex | υ Ce-O des Clustergerüsts |

11. Verfahren zur Herstellung einer Cer(lV)-Verbindung nach einem der Ansprüche 1 bis 10, bei dem ein Cer(lV)-Salz mit einem Salz einer organischen Säure mit einem pKa-Wert größer als 1 oder mit einer Mischung aus Salzen organischer Säuren, worunter mindestens eine einen pKa-Wert größer als 1 aufweist, vermischt wird, wobei mindestens 2 Mol-Teile des genannten Salzes einer organischen Säure je Cer-Atom eingesetzt werden.

12. Verfahren nach Anspruch 11, bei dem das Cer(IV)-Salz ein Cer(IV)-Ammoniumnitrat ist.

13. Verfahren nach Anspruch 11 oder 12, bei dem die Mischung in einem wäßerigen Medium erfolgt.

14. Lösung einer Cer(lV)-Verbindung nach einem der Ansprüche 1 bis 10 in einer Carbonsäure oder in einem Kohlenwasserstoff-Lösungsmittel.

15. Filmbildende Zusammensetzung, die unter Einwirkung der atmosphärischen Oxidation austrocknet und eine Cer(lV)-Verbindung nach einem der Ansprüche 1 bis 10 als Oxidationskatalysator enthält.

16. Kohlenwasserstoff-Brennstoff, der eine öllösliche Cer(lV)-Verbindung nach einem der Ansprüche 1 bis 10 enthält.

17. Kraftstoff nach Anspruch 16 für Verbrennungsmotoren, insbesonders Kraftstoff für Dieselmotoren.

## Revendications

1. Composé de cérium-IV comportant, pour chacun des atomes de Ce-IV, deux radicaux d'un acide organique ayant un pKa supérieur à 1, ou d'un de mélange de tels acides, et un atome d'oxygène lié audit atome de Ce-IV en plus des atomes d'oxygène présents dans lesdits radicaux d'acide organique, et pouvant être obtenu par réaction d'un sel de cérium-lV avec un sel dudit acide organique ayant un pKa supérieur à 1 ou avec un mélange de sels d'acides organiques dont au moins un présente un pKa supérieur à 1, auquel cas on met en oeuvre au moins deux parties molaires desdits sels de l'acide organique par atome de cérium.

2. Composé de cérium-lV de formule :
(H₂O)ₚ[CeO(A)₂.(AH)ₙ.]ₘ
dans laquelle les radicaux A sont identiques ou différents et représentent chacun le radical d'un acide organique de formule AH, tel que défini dans la revendication 1, p est un nombre entier de 0 à 5, n est compris entre 0 et 2 et m est un nombre entier de 1 à 12.

3. Composé de cérium-lV selon la revendication 2 de formule :
(H₂O)₄[CeO(A)₂.(AH)ₙ.]₆
dans laquelle A est défini dans la revendication 2 et n est égal à 0 ou 2.

4. Composé de cérium-lV selon les revendications 1, 2 ou 3 dans lequel ledit pKa est supérieur à 2.

5. Composé de cérium-lV selon l'une des revendications 1 à 4 dans lequel l'acide organique est un acide carboxylique, un acide organique sulfurique, sulfonique, phosphorique ou phosphonique.

6. Composé de cérium-lV selon l'une des revendications 1 à 5 dans lequel l'acide organique est un acide monocarboxylique ayant de 2 à 20 atomes de carbone ou un acide dicarboxylique ayant de 4 à 12 atomes de carbone.

7. Composé de cérium-lV selon l'une des revendications 1 à 5 dans lequel l'acide organique est un acide alcanoïque ayant de 4 à 12 atomes de carbone, un acide benzoïque ou un acide benzoïque substitué par un alkyle ayant jusqu'à 12 atomes de carbone ou un de leurs mélanges.

8. Composé de cérium-lV selon les revendications 1, 2 ou 3 dans lequel l'acide organique est un ou plusieurs des acides suivants : acide acétique, acide propionique, acide butyrique, acide méthyl-2 propionique, acide pivalique, acide méthyl-2 butyrique, acide éthyl-2 butyrique, acide méthyl-2 pentanoïque, acide éthyl-2 hexanoïque, acide diméthyl-3,5 hexanoïque, acide néohexanoïque, acide néoheptanoïque, acide néooctanoïque, acide isononanoïque, acide néodécanoïque, acide undécylénique, acide naphténique, acide cyclohexane carboxylique, acide benzoïque, acide p-tert-butyl-benzoïque, acide maléique, acide sébacique, acide bis(2-éthylhexyl)phosphorique, acide docécylbenzène sulfonique et acide dodécylsulfurique.

9. Composé de cérium-lV de formule :
H₆Ce₆O₈(OH)₂(RCOO)₁₂
dans laquelle R est un alkyle ayant de 3 à 9 atomes de carbone,
de structure octaédrique, les six atomes de cérium étant situés aux sommets de l'octaèdre, les douze radicaux carboxylate formant des pontages bidentaux entre les atomes de cérium le long des arêtes de l'octaèdre, un atome d'oxygène formant un triple pontage sur chacune des faces de l'octaèdre et deux ligands hydroxyle unidentaux complétant la coordination de deux atomes de cérium opposés.

10. Composé de cérium-lV selon l'une des revendications précédentes présentant les principales absorptions IR indiquées dans le Tableau ci-dessous :
| BANDE | ATTRIBUTIONS PROPOSEES |
|---|---|
| 1. 3650 cm⁻¹ forte, très effilée | υ OH ? ν Ce-O-OH ou OH isolé de l'acide carboxylique |
| 2. 3400-2400 cm⁻¹ forte, très large | υ OH de l'acide carboxylique libre |
| 3. 1750-1720 cm⁻¹ très forte, très effilée | υ C=O de l'acide carboxylique |
| 4. 1580-1550 cm⁻¹ très forte, très effilée | υ C=O du µ, η carboxylate |
| 5. 700-500 cm⁻¹ faible, complexe | υ Ce-O du squelette de l'agrégat |

11. Procédé de préparation d'un composé de cérium-lV tel que défini dans l'une des revendications 1 à 10 comportant le mélange d'un sel de cérium-lV avec un sel d'un acide organique ayant un pKa supérieur à 1 ou avec un mélange de sels d'acides organiques dont l'un au moins a un pKa supérieur à 1 auquel cas on met en oeuvre au moins 2 parties molaires dudit sel d'un acide organique par atome de cérium.

12. Procédé selon la revendication 11 dans lequel le sel de cérium-lV est un nitrate d'ammonium cérique.

13. Procédé selon les revendications 11 ou 12 dans lequel le mélange est effectué dans un milieu aqueux.

14. Solution de composé de cérium-lV tel que défini dans l'une des revendications 1 à 10 dans un acide carboxylique ou un solvant hydrocarboné.

15. Composition filmogène séchant sous l'effet de l'oxydation atmosphérique comportant comme catalyseur d'oxydation un composé de cérium-lV tel que défini dans l'une des revendications 1 à 10.

16. Combustible hydrocarboné comportant un composé de cérium-lV oléosoluble tel que défini dans l'une des revendications 1 à 10.

17. Carburant selon la revendication 16 destiné aux moteurs à combustion interne, en particulier carburant pour moteurs diesel.
